# FASCICULE DE BREVET EUROPEEN

(11) **EP 2 035 556 B1**
(45) Date de publication et mention de la délivrance du brevet: **29.07.2015**
(21) Numéro de dépôt: 06764790.9
(22) Date de dépôt: 15.06.2006
(51) Int. Cl.: A61K 38/00, C12N 9/20, C07K 14/39, C12P 21/02

(54) **PROCEDE DE PRODUCTION DE LIPASE, CELLULE DE YARROWIA LIPOLYTICA TRANSFORMEE APTE A PRODUIRE LADITE LIPASE ET LEURS APPLICATIONS**
VERFAHREN ZUR HERSTELLUNG VON LIPASE, TRANSFORMIERTE YARROWIA LIPOLYTICA-ZELLE MIT FÄHIGKEIT ZUR HERSTELLUNG DER LIPASE UND VERWENDUNGEN DAVON
METHOD FOR PRODUCING LIPASE, TRANSFORMED YARROWIA LIPOLYTICA CELL CAPABLE OF PRODUCING SAID LIPASE AND THEIR USES

(43) Date de publication de la demande: 18.03.2009
(73) Titulaire: Laboratoires Mayoly Spindler, 78400 Chatou (FR)
(72) Inventeur: LEBLOND, Yves, 78630 Orgeval (FR); MOUZ, Nicolas, 38760 St Paul de Varces (FR); MARTY, Alain, 31400 Toulouse (FR); URIBELARREA, Jean-Louis, 31400 Toulouse (FR)
(74) Mandataire: Leblois-Préhaud, Hélène Marthe Georgette
(86) Numéro de dépôt international: PCT/FR2006/001352
(87) Numéro de publication internationale: WO 2007/144475

(56) Documents cités:
- WO-A-01/83773
- WO-A2-2006/039541
- ALONSO F.O.M. ET AL.: "Improvement of lipase production ..." BRAZILIAN JOURNAL OF CHEMICAL ENGINEERING, vol. 22, no. 1, mars 2005 (2005-03), pages 9-18, XP002414756
- FICKERS PATRICK ET AL: "Methyl oleate modulates LIP2 expression in the lipolytic yeast Yarrowia lipolytica" BIOTECHNOLOGY LETTERS, vol. 27, no. 22, novembre 2005 (2005-11), pages 1751-1754, XP002414757 ISSN: 0141-5492
- CAMARGO-DE-MORAIS M M ET AL: "Oil/mineral-salts medium designed for easy recovery of extracellular lipase from Fusarium oxysporumn AM3.", WORLD JOURNAL OF MICROBIOLOGY AND BIOTECHNOLOGY, vol. 19, no. 1, February 2003 (2003-02), pages 17-20, ISSN: 0959-3993
- PEZZILLI RAFFAELE: "Chronic pancreatitis: maldigestion, intestinal ecology and intestinal inflammation.", WORLD JOURNAL OF GASTROENTEROLOGY : WJG 14 APR 2009 LNKD- PUBMED:19360910, vol. 15, no. 14, 14 April 2009 (2009-04-14), pages 1673-1676, ISSN: 1007-9327
- WALJEE A K ET AL: "Systematic review: pancreatic enzyme treatment of malabsorption associated with chronic pancreatitis", ALIMENTARY PHARMACOLOGY & THERAPEUTICS, vol. 29, no. 3, February 2009 (2009-02), pages 235-246, ISSN: 0269-2813

## Description

La présente invention est relative à un procédé de production de lipase mettant en oeuvre des cellules de levure *Yarrowia lipolytica,* productrices d'une lipase recombinante acido-résistante, lequel procédé permet l'obtention d'une lipase apte à être mise en oeuvre comme médicament ; la présente invention est également relative à une souche de *Yarrowia lipolytica* surproductrice de lipase recombinante acido-résistante et à ses applications.

Les aliments ingérés quotidiennement par l'homme sont constitués essentiellement de lipides, de protéines et de sucres. Tous ces constituants subissent, avant leur absorption, une hydrolyse catalysée par les enzymes du tractus digestif. Un déficit en l'une ou l'autre de ces enzymes peut donc entraîner des troubles digestifs, et conduire à une malnutrition importante. C'est par exemple le cas dans certaines situations pathologiques, comme la mucoviscidose ou l'insuffisance pancréatique exocrine, auxquelles est associé un déficit en lipase pancréatique. Il est classiquement proposé pour corriger ce déficit, d'administrer par voie orale des extraits pancréatiques. Toutefois l'efficacité de cette thérapeutique est limitée par le fait que les enzymes contenues dans ces extraits (lipases, amylases et protéases) sont rapidement inactivées par l'acidité du milieu gastrique.

Il a donc été proposé d'utiliser des préparations de lipases qui résistent aux conditions gastriques, telles que par exemple des préparations de lipase gastrique de mammifères produites par génie génétique, comme celle décrite dans la Demande de Brevet Français publiée sous le numéro 2 699 179, au nom de l'INSTITUT DE RECHERCHE JOUVENIAL SA ou des préparations de lipases de microorganismes possédant une activité correcte en milieu acide [ZENTLER-MONRO et al., Pancreas, 7, 311-319 (1992)].

Parmi les lipases de microorganismes actives en milieu acide, on citera en particulier des lipases fongiques, telles que celles de *Candida ernobii*, [YOSHIDA et al., Biochim. Biophys. Acta,; 154, 586-588, (1968)], de *Trichosporon asteroide* [DHARMSTHITI et al., Biotechnol. Appl. Biochem., 26, 111-116 (1997)], de *Rhizopus javanicus* [(UYTTENBROECK et al., Biol. Chem. Hoppe Seyler, 374, 245-254, (1993)], ou de *Yarrowia lipolytica* [HADEBALL, Acta Biotechnol., 2, 159-167 (1991) ; NOVOTNY et al., J. Basic Microbiol., 28, 221-227 (1988)]. Outre leur activité à pH acide, ces lipases présentent les caractéristiques communes d'être résistantes, en présence de leur substrat, à la digestion par les protéases (trypsine, chymo-trypsine et pepsine), et d'être résistantes à l'action des sels biliaires (leur activité est conservée en présence de 10 mM de taurocholate de sodium).

L'utilisation de *Yarrowia lipolytica* pour la production d'un gène d'intérêt a déjà été décrite. Ainsi, la Demande EP 1 108 043, aux noms de l'INRA et du CNRS, décrit l'utilisation d'un vecteur intégratif, comprenant une cassette d'expression d'un gène d'intérêt et des séquences zêta, correspondant aux séquences LTR du rétrotransposon Ylt de *Yarrowia lipolytica.* Un tel vecteur d'expression permet l'intégration non homologue et dispersée de plusieurs copies de l'insert d'intérêt dans l'ADN génomique d'une souche de *Yarrowia lipolytica* dépourvue de séquences zêta. Ce système a notamment été utilisé pour l'intégration du gène *LIP2,* codant pour une lipase, dans l'ADN de *Yarrowia lipolytica* et a permis, dans des conditions de culture qui ne sont pas détaillées, une sécrétion de lipase 10 à 15 fois supérieure à celle des souches non transformées.

D'autres études décrivent l'utilisation du même vecteur d'expression que celui décrit dans la Demande de Brevet EP 1 108 043, pour la production d'une lipase recombinante chez *Yamowia lipolytica* (Demande Internationale WO 01/83773 ; PIGNEDE et al., Journal of Bacteriology, Vol 182, No.10, p.2802-2810 (2000) et PIGNEDE et al., Applied and Environmental Microbiology, Vol. 66, No.8, p3283-3289 (2000)). Ainsi :
- la Demande Internationale WO 01/83773, au nom des Laboratoires MAYOLY SPINDLER, décrit l'obtention du clone de *Yarrowia lipolytica* MS4 (CNCM I-2294), comprenant 10 copies de la cassette d'expression du gène *LIP2* intégrées dans son ADN, ainsi que son utilisation pour la production de lipase avec un rendement de l'ordre de 0,5 g de lipase par litre de surnageant de culture, et une activité catalytique de 12 000 U/ml, mesurée en utilisant l'huile d'olive comme substrat, une Unité correspondant à la quantité d'enzyme capable de catalyser la libération de 1 µmole d'acide gras par minute, c'est-à-dire 200 fois plus que la souche initiale. Cependant, le procédé de production de lipase décrit dans cette Demande présente un inconvénient majeur, puisque il met en oeuvre des milieux de culture contenant du bactopeptone ou du bactotryptone. Ces produits, qui ne sont pas caractérisés et qui contiennent divers hydrolysats protéiques, sont classiquement utilisés comme source d'azote et de carbone. En conséquence, le procédé décrit dans cette Demande ne permet pas d'obtenir une lipase directement utilisable comme médicament.
- PIGNEDE et al. (Journal of Bacteriology, 2000) ont plus particulièrement caractérisé la lipase extracellulaire codée par le gène *LIP2* de *Yarrowia lipolytica* (souche POld). Dans cet article sont ainsi étudiées :
   - la sécrétion de la lipase à partir de différentes souches sauvages (POld dans laquelle Yltl est absent et E150, dans laquelle Yltl est présent) et de différentes souches recombinantes dont JMY184 (POld-6-15) et JMY279 (POld-6-17) ainsi que
   - la surproduction de lipase notamment par le transformant JMY184.

   Cet article de PIGNEDE *et al.* compare la production de lipase par des souches sauvages, des souches mutantes et des souches recombinantes obtenues selon le procédé décrit plus haut (Demande Internationale WO 01/83773). Les souches sauvages secrètent entre 30 et 50 U de lipase/ml alors que des souches mutantes, obtenues par l'action du N-méthyl-N'nitro-N-nitrosoguanidine (NNNG), produisent 25 fois plus de lipase, c'est-à-dire 1 200 U/ml dans des conditions de culture optimisées faisant intervenir un milieu contenant du peptone (milieu de préculture) et un milieu contenant du lactosérum (milieu de fermentation) (voir également DESTAIN *et al.,* 1997). Les souches recombinantes sont obtenues à l'aide de la construction comprenant le gène *LIP2* régulé par le promoteur POX2 et par l'intégration non homologue, en multicopies et de façon dispersée, de cette cassette d'expression. PIGNEDE *et al.* ont obtenu des transformants stables (par exemple la souche JMY184) qui produisent 2 000 U/ml dans des conditions non optimisées, c'est-à-dire en milieu YPDH (comprenant 10g/l d'extrait de levure, 10g/l de bactopeptone, 10g/l de glucose et 10g/l d'huile d'olive) correspondant à environ 0,5 g de lipase/l de surnageant. De la même manière que précédemment, les préparations de lipase décrites par PIGNEDE *et al.* et par DESTAIN *et al.,* sont impropres, en tant que telles, à un usage médical et plus particulièrement à la constitution de lots cliniques, puisque leur production nécessite l'utilisation de milieux de culture contenant des peptones ou du lactosérum.
- Poursuivant ses travaux, l'équipe de PIGNEDE (Applied and Environmental Microbiology, 2000) a étudié des souches de *Yarrowia lipolytica* transformées par un vecteur comprenant une cassette d'expression du gène *LIP2.* Les Auteurs ont déterminé que pour 8 de ces transformants, le nombre de copies de la cassette d'expression du gène *LIP2* est compris entre 6 et 16 (10 copies en moyenne), ce qui se traduit par 2 à 15 évènements d'intégration de ladite cassette à des loci différents. La souche JMY184 comprend ainsi 12 copies de la cassette d'expression du gène *LIP2,* intégrées à 4 loci différents. Les Auteurs ont par ailleurs plus précisément étudié ce transformant JMY184. Ils confirment que la souche JMY 184 produit 0,5 g de lipase/l de surnageant avec une activité de 1 500 U/ml sur milieu riche YPDH (qui contient du bactopeptone) (contre 50 U/ml pour une souche sauvage POld), mesurée en utilisant l'huile d'olive comme substrat. Ces valeurs permettent de déduire une activité spécifique d'environ 3 000 U/mg de lipase. Les Auteurs annoncent en outre que la production optimisée de lipase en fermenteur par la souche JMY184 permet d'obtenir des préparations ayant une activité jusqu'à 10 000 U/ml. Cependant les conditions de culture qui ont permis ce résultat ne sont pas divulguées. Dans cet article, les Auteurs ont en outre étudié la stabilité en culture de ces transformants, et notamment du clone JMY184, et ont montré leur stabilité pendant 120 générations. PIGNEDE *et al.* ont considéré que pour optimiser la production de lipase, les facteurs à prendre en compte sont la stabilité des transformants et les conditions de culture.

Par ailleurs, ils ont montré qu'il existe une forte corrélation entre le nombre de copies du gène *LIP2* intégrées et la surproduction de lipase.

Cependant, pour la production de lipase, les seuls milieux de culture envisagés dans cet article sont des milieux qui contiennent des peptones, tels que le milieu riche YPDH.

Les procédés de production de lipase de l'art antérieur, même s'ils permettent d'obtenir des rendements améliorés en lipase, ne sont pas adaptés à la production d'une lipase apte à être utilisée à des fins médicales.

En effet, les milieux de culture habituellement mis en oeuvre contiennent tous des mélanges non caractérisés et/ou des produits d'origine animale, tels que des peptones, des tryptones ou du lactosérum. Il existe donc un besoin de développer un système permettant la production de préparations de lipase recombinante adaptée à un usage médical.

Pour résoudre ce problème, les Inventeurs ont mis au point un procédé de production de lipase qui répond mieux à ces besoins que les procédés de production de l'art antérieur. Plus précisément, les Inventeurs ont mis au point un procédé de production de lipase dans lequel le milieu de culture mis en oeuvre est dépourvu des produits précités, c'est-à-dire qu'il ne contient aucun produit d'origine animale ni aucun mélange non caractérisé, tel que le peptone, le tryptone ou le lactosérum. Les Inventeurs ont en outre sélectionné une nouvelle souche de *Yarrowia lipolytica* recombinante productrice de la lipase Lip2, qui, associée audit procédé, permet en outre d'augmenter de manière significative, le rendement en lipase produite.

La présente invention a donc pour objet un procédé de production de lipase, mettant en oeuvre une souche de *Yarrowia lipolytica* transformée par un vecteur comprenant une cassette d'expression d'une lipase acido-résistante de levure, caractérisé en ce que le milieu de culture utilisé ne contient pas de produits d'origine animale ni de mélanges non caractérisés, tel que le peptone, le tryptone ou le lactosérum.

Plus précisément, la présente invention a pour objet un procédé de production de lipase recombinante, comprenant :
a) une étape de mise en culture de cellules de *Yarrowia lipolytica* transformées par un vecteur d'expression comprenant une cassette d'expression d'une lipase acido-résistante de levure, dans des conditions permettant la production de lipase ;
b) une étape de récupération, au cours de laquelle on recueille, dans le surnageant de ladite culture, de la lipase recombinante ainsi produite par lesdites cellules ;
lequel procédé est caractérisé en ce que l'étape a) de mise en culture est mise en oeuvre dans un milieu de culture dépourvu de produits d'origine animale ou de mélanges non caractérisés constitués de matières protéiques d'origine animale (lactosérum par exemple) ou de leurs produits de digestion enzymatique (tryptone, ou peptone par exemple)
et en ce que ladite étape a) comprend :
al) la pré-culture des cellules transformées de *Yarrowia lipolytica* dans un milieu contenant une source de carbone d'origine glucidique, de l'azote minéral, de préférence du sulfate d'ammonium, et sels minéraux, des oligoéléments et des vitamines ; et
a2) une étape de fermentation, comprenant une phase de croissance cellulaire dans un milieu contenant comme seule source de carbone une source de carbone d'origine glucidique, de l'azote minéral et sels minéraux, des oligoéléments et des vitamines et une phase de production de lipase dans un milieu contenant comme seule source de carbone l'acide oléique, de l'azote minéral et sels minéraux, des oligoéléments et des vitamines.

La fermentation est avantageusement mise en oeuvre avec une pO2 constante comprise entre 15 % et 25 % et un pH de préférence inférieur à 6,5. La fermentation peut par exemple être mise en oeuvre avec un débit d'air d'environ 1 vvm, une unité vvm étant 1 volume d'air par volume de liquide par minute (par exemple pour un fermenteur de 30 litres, 1 vvm est égal à 30 1/min et pour un fermenteur de 5 litres, 1 vvm est égal à 5 1/min).

Selon une disposition avantageuse de ce mode de mise en oeuvre, l'étape al) de pré-culture est réalisée jusqu'à atteindre une valeur de DO₆₀₀ₙₘ comprise entre 3 et 10 pour 1 ml, et à l'étape a2) de fermentation, ladite phase de synthèse de lipase est initiée lorsque la DO₆₀₀ₙₘ de la culture atteint une valeur comprise entre 60 et 70 pour 1 ml.

Selon une autre disposition avantageuse de ce mode de mise en oeuvre, l'étape b) est initiée lorsque la DO₆₀₀ₙₘ atteint une valeur comprise entre 300 et 350 par ml. L'étape b) peut en outre comprendre :
b1) la séparation de la lipase dudit surnageant de culture ;
b2) la purification de la lipase obtenue en b1).

Ces deux étapes sont réalisées par des techniques classiques connues en elles-mêmes : séparation physique (filtration, chromatographie, centrifugation) ou physico-chimique (précipitation).

La séparation de la lipase du surnageant peut être mise en oeuvre par l'homme du métier par exemple par une technique choisie parmi la filtration tangentielle en fibre creuse, la filtration frontale et la centrifugation continue ou discontinue.

La purification de la lipase consiste notamment à réduire la biocharge, c'est-à-dire la présence de microorganismes, et peut être mise en oeuvre par toute technique de purification adéquate connue de l'homme du métier, telle qu'une technique choisie parmi la filtration, la précipitation fractionnée, la chromatographie d'échange d'ions, la chromatographie d'interactions hydrophobes et la chromatographie par gel-filtration.

Optionnellement, le procédé de production de lipase selon l'invention peut également comprendre une étape de concentration, ou enrichissement, consistant en l'augmentation de la concentration en lipase dans la préparation. Une telle étape peut être mise en oeuvre par exemple après l'étape de purification. Elle peut également avoir lieu simultanément à cette étape de purification.

Le procédé de production selon l'invention permet notamment de produire la lipase recombinante en quantités situées entre environ 1 à 3 g de lipase purifiée par litre de surnageant de culture. De manière particulièrement avantageuse, le procédé selon l'invention permet l'obtention d'une préparation de la lipase recombinante codée par le gène Lip2 ou LIP2 avec une activité catalytique supérieure à 15000 U par ml de surnageant de culture. De manière préférée, ladite préparation possède une activité supérieure à 20000 U/ml de surnageant de culture. Ces valeurs sont déterminées à pH 6, en utilisant la trioctanoïne comme substrat. Cette valeur d'activité de la préparation de lipase revêt un intérêt tout particulier dans le cadre du développement de produits pharmaceutiques. Il est en effet maintenant envisageable d'administrer des doses réduites de lipase, produite par le procédé selon l'invention, tout en gardant une efficacité suffisante pour obtenir l'effet thérapeutique désiré, qui peut être par exemple la correction d'une malabsorption de graisse liée à une insuffisance pancréatique associée à un déficit en lipase.

Le fait de pouvoir désormais produire la lipase Lip2 en utilisant des souches de *Yarrowia lipolytica* recombinantes sans faire appel à des produits d'origine animale, comme c'est le cas avec le procédé selon l'invention, constitue un avantage considérable et facilite grandement l'utilisation de la lipase à des fins médicales.

Le vecteur d'expression utilisé pour obtenir les cellules de *Yarrowia lipolytica* recombinantes utilisées dans le procédé de la présente invention est un vecteur intégratif possédant au moins une copie du gène *LIP2* et des éléments de régulation de l'expression. Ce vecteur peut être alors intégré soit dans l'ADN plasmidique soit dans l'ADN génomique de la levure. Un exemple de vecteur intégratif particulièrement préféré est le vecteur JMP6 ou le vecteur JMP10 tels que décrits dans la Demande Internationale WO01/83773. Le vecteur JMP6 comprend une cassette d'expression du gène *LIP2,* codant pour le précurseur Lip2p de la lipase Lip2 de *Yarrowia lipolytica,* en amont duquel est placé le promoteur de l'acyl CoA oxydase ACO2 de *Yarrowia lipolytica,* dénommé POX2. Le vecteur JMP10 comprend également le gène *LIP2,* en amont duquel se trouve le promoteur de la lipase Lip2 de *Yarrowia lipolytica.* Ces deux promoteurs sont inductibles par les triglycérides et les acides gras. Dans chacun de ces vecteurs, la cassette d'expression et le promoteur sont placés entre des séquences zêta comme décrit plus haut. L'intégration peut être ciblée, c'est-à-dire dirigée sur des sites, ou bien aléatoire. Ainsi, lorsque la souche de *Yarrowia lipolytica* transformée est dépourvue de séquence zêta (c'est par exemple le cas pour la souche POld), l'intégration de la cassette d'expression est dispersée dans l'ADN génomique de ladite souche. En revanche, lorsque la souche de *Yarrowia lipolytica* comporte des séquences zêta (c'est par exemple le cas de la souche E150), l'intégration est principalement ciblée au niveau de ces séquences.

Selon un autre mode de mise en oeuvre avantageux dudit procédé, la souche transformée de *Yarrowia lipolytica* est de préférence la souche dénommée YL-LIP2-6C, déposée auprès de la Collection Nationale de Cultures de Microorganismes (C.N.C.M.), 28 rue du Docteur Roux, 75724 Paris Cedex 15, le 15 décembre 2005, sous le numéro I-3542. Cette souche YL-LIP2-6C est génétiquement stable.

Au sens de la présente invention, les expressions « stable » « génétiquement stable », « stabilité génétique », ainsi que leurs variantes, sont utilisées en référence à la conservation d'un même nombre de loci d'intégration d'un acide nucléique d'intérêt dans l'ADN du clone considéré, pendant au moins 30 générations. Ce nombre de 30 générations est choisi comme base de calcul de la stabilité génétique car les inventeurs ont constaté qu'il correspond à un nombre de doublements de la population cellulaire suffisant pour permettre la production de lipase en utilisant un procédé comprenant au moins une étape de pré-culture de cellules *Yarrowia lipolytica* recombinantes et une étape de production de lipase proprement dite en condition de fermentation. Au sens de la présente invention, une génération est définie par le doublement de la population cellulaire et peut être calculée selon la formule Y = X*2^{g} où Y est la population cellulaire au temps t (par exemple exprimée en densité cellulaire), X est la population cellulaire initiale au temps t₀ et g représente le nombre de générations nécessaires pour que la population cellulaire passe de la valeur X à la valeur Y. De manière préférée, le clone est dit génétiquement stable lorsque au moins 90% des colonies analysées, après au moins 30 générations, contiennent le même nombre de loci du gène d'intérêt que le clone de départ. Ainsi, comme il ressort des exemples, le clone YL-LIP2-6C est dit stable car près de 100% des colonies analysées après 100 générations contiennent 6 loci du gène *LIP2* (un locus correspondant au gène *LIP2* endogène + 5 loci d'intégration de la cassette d'expression du gène *LIP2*)*.*

De manière surprenante, lorsque le procédé selon l'invention met en oeuvre cette souche particulière, les Inventeurs ont réussi à produire la lipase recombinante en grande quantité et à avoir un meilleur contrôle sur ladite production. Les Inventeurs ont en effet réussi à améliorer le rendement de production de lipase et ont notamment pu obtenir une production de l'ordre de 1 à 3 g de lipase pure par litre de surnageant de culture. Ils ont également pu obtenir des préparations de lipase présentant une activité proche de 20 000 U/ml.

Au sens de la présente invention, l'activité de la lipase correspond à son activité enzymatique. L'activité catalytique d'une solution de lipase est exprimée en unité (U) par ml de solution analysée. L'activité spécifique est exprimée en unité (U) par mg de protéine (lipase) purifiée. Une unité correspond à la quantité d'enzyme capable de catalyser la libération de 1 µmole d'acide gras par minute. L'activité spécifique d'une lipase varie en fonction de la nature du triglycéride utilisé comme substrat.

Outre l'amélioration du rendement de production de la lipase, les Inventeurs ont également pu assurer une meilleur reproductibilité du procédé de production, améliorer l'homogénéité du produit final et améliorer les conditions de purification de la lipase.

Ces différentes modifications permettent donc d'obtenir une lipase répondant aux conditions requises pour un usage médical. En effet, dans le cadre de leur recherche, les Inventeurs ont maintenant observé que la lipase Lip2 est active dans des valeurs de pH supérieures à 3 et jusqu'à une valeur de pH proche de 8, avec une activité optimale pour un pH compris entre 5 et 6. En revanche, elle est inactivée de manière irréversible lors d'une incubation de 2 heures à pH3 ou pH8,5. L'activité spécifique de la lipase Lip2 a également été analysée en fonction de différents substrats et les Inventeurs ont ainsi déterminé à pH4 les valeurs d'environ 10 760 U/mg, environ 16 920 U/mg et environ 12 260 U/mg de lipase purifiée, respectivement avec des triglycérides à chaîne courte (tributyrine), moyenne (trioctanoïne) et longue (huile d'olive). Enfin, les Inventeurs ont constaté de manière surprenante que la lipase Lip2 est active en présence de sels biliaires et que cette activité augmente avec la concentration en sels biliaires. Cette activité en présence de sels biliaires avait jusqu'à présent seulement été observée avec la lipase gastrique et la lipase pancréatique associée à la co-lipase. Ainsi l'utilisation de lipase Lip2 avec une importante activité spécifique ne nécessite pas la présence de co-lipase.

Le clone YL-LIP2-6C contient plusieurs copies de la cassette d'expression de cette lipase Lip2 se traduisant par 5 évènements d'intégration du gène *LIP2* à des loci différents. Lorsqu'il est mis dans des conditions adaptées à la production de lipase, le clone YL-LIP2-6C produit davantage de lipase que les souches transformées de *Yarrowia lipolytica* de l'art antérieur. La lipase est produite selon un rendement supérieur à 1 g/l de surnageant de culture, c'est-à-dire supérieur au rendement observé avec les clones de l'art antérieur, décrits plus haut.

La présente invention a également pour objet une préparation de lipase susceptible d'être obtenue par le procédé selon l'invention.

Selon un mode de réalisation avantageux de ladite préparation de lipase, elle présente une activité au moins égale à 15000 U/ml, de préférence supérieure à 20000 U/ml lorsqu'elle est déterminée comme indiqué plus haut.

L'invention a en outre pour objet l'utilisation de la préparation de lipase selon l'invention pour la préparation d'un médicament destiné au traitement d'une pathologie associée à un dérèglement de l'absorption des graisses (par exemple acides gras à chaînes courtes, moyennes ou longues), notamment lié à une insuffisance pancréatique, notamment exocrine.

L'invention a également pour objet un médicament, caractérisé en ce qu'il comprend une préparation de lipase telle que définie ci-dessus.

La présente invention a également pour objet une cellule de *Yarrowia lipolytica* transformée par un vecteur d'expression d'une lipase acido-résistante de levure, caractérisée en ce qu'il s'agit du clone dénommé YL-LIP2-6C, déposé auprès de la Collection Nationale de Cultures de Microorganismes (C.N.C.M.), 28 rue du Docteur Roux, 75724 Paris Cedex 15, le 15 décembre 2005 sous le numéro I-3542.

La présente invention a, en outre, pour objet l'utilisation d'une cellule telle que définie ci-dessus pour la production de la lipase acido-résistante de levure codée par le gène Lip2 ou LIP2.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

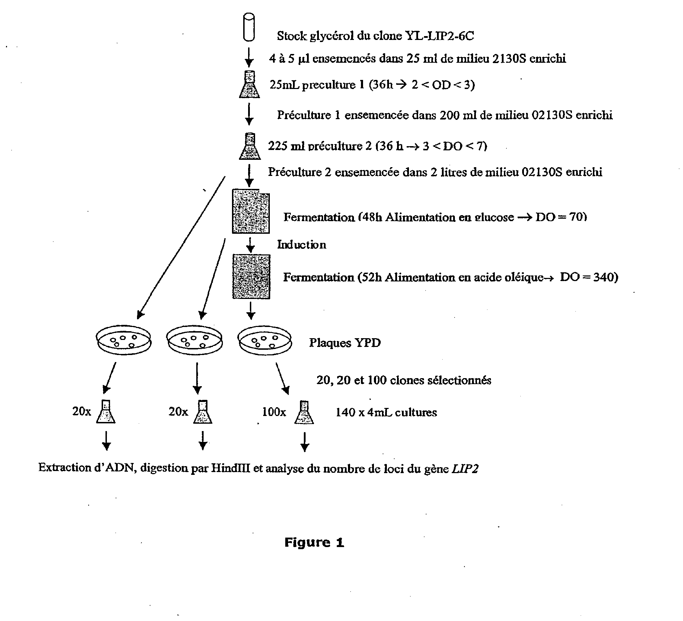

- La figure 1 représente l'approche globale du contrôle de la stabilité génétique du clone YL-LIP2-6C au cours du procédé de production de lipase.

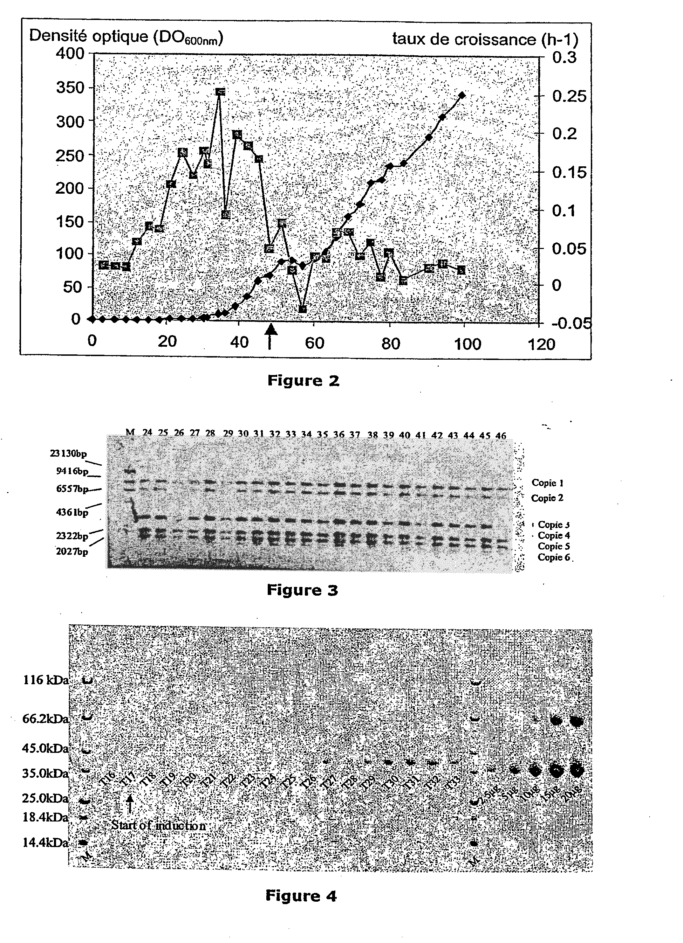

- La figure 2 représente la variation de la densité optique à 600 nm (DO₆₀₀, axe des ordonnées gauche) (-◆-) et la variation du taux de croissance (h⁻¹, axe des ordonnées droit) (-■-) en fonction du temps (heures, axe des abscisses) durant le procédé de fermentation. La flèche indique l'induction de la production de lipase.
- La figure 3 représente l'analyse par Southern blot du nombre d'événements d'intégration du gène *LIP2* à des loci différents dans le génome de 23 colonies (pistes 24 à 43) prélevées au temps T33, correspondant à la fin de la fermentation, environ 100 heures après le début de la fermentation. Copies 1 à 6 : 6 loci du gène *LIP2* (5 loci pour l'intégration de la cassette d'expression du gène *LIP2 +* un locus pour le gène *LIP2* endogène). M : marqueur de taille.
- La figure 4 représente le suivi, par SDS-PAGE, de la production de lipase recombinante par le clone YL-LIP2-6C pendant le procédé de production, de T16 à T33. La flèche indique l'induction de la production de lipase (T17, 48 heures de fermentation). M : échelle de poids moléculaire ; les cinq pistes de la partie droite du gel correspondent à des quantités connues (2,5 µg à 20 µg) de la lipase Lip2 purifiée.

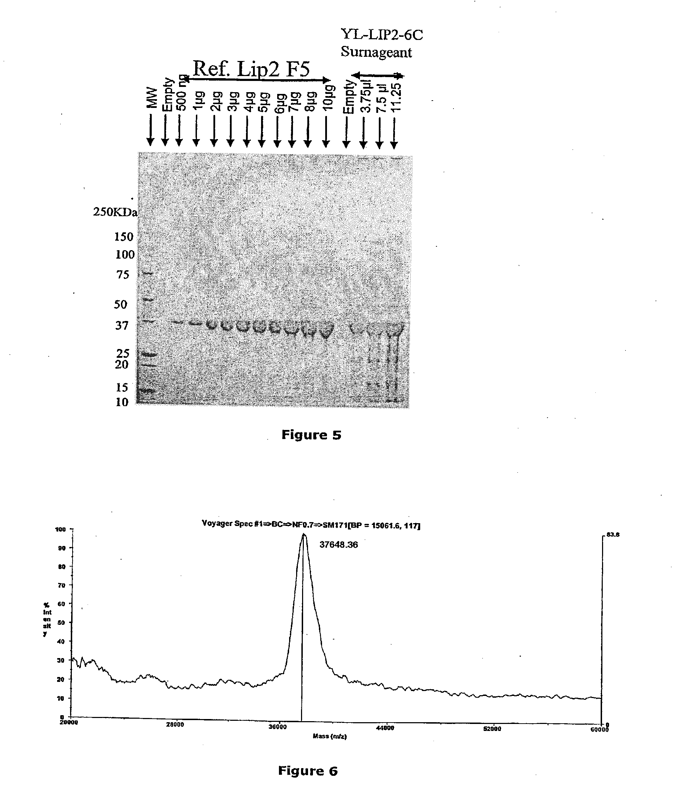

- La figure 5 représente l'analyse, par SDS-PAGE, de la pureté de la lipase dans le surnageant au temps T33 (fin du procédé de fermentation). MW : échelle de poids moléculaire ; Ref.Lip2 F5 : gamme de lipase Lip2 de 1 à 10 µg ; YL-LIP2-6C surnageant : volumes, en µl, du surnageant analysé.
- La figure 6 représente le spectre de masse, déterminé par spectrométrie de masse de type MALDI-TOF, de la lipase recombinante produite par le clone YL-LIP2-6C.

### EXEMPLE 1- MATERIELS ET METHODES

### 1) Milieux utilisés

Les concentrations finales indiquées sont les concentrations dans les solutions-mères.
Milieu de base 02130S non enrichi

| **Ingrédients** | **concentration finale** |
|---|---|
| Glucose | 10 g/l |
| KH₂PO₄ | 3 g/l |
| Na₂HPO₄ | 3 g/l |
| H₃BO₃ | 0,34 g/l |
| (NH₄)₂SO₄ | 3 g/l |
| C₅H₈O₄NNa (Glutamate) | 1 g/l |
| MgSO₄ | 0,5 g/l |
| CaCl₂ | 0,023 g/l |
| MnSo₄ | 0,038 g/l |
| ZnSO₄ | 0,04 g/l |

Le milieu 02130S enrichi comprend en outre les additifs suivants :

| **Additifs** | **Concentration finale** |
|---|---|
| Solution d'oligoéléments | 1 ml/l |
| Solution de vitamines | 1 ml/l |

Composition de la solution d'oligoéléments

| **Ingrédients** | **Concentration finale** |
|---|---|
| CoCl₂ | 0,5 g/l |
| Na₂MoO₄ | 0,06 g/l |
| CuSO₄ | 0,9 g/l |

Composition de la solution de vitamines

| **Ingrédients** | **Concentration finale** |
|---|---|
| D-biotine | 0,05 g/l |
| Pantothénate de calcium | 1 g/l |
| Acide nicotinique | 1 g/l |
| Myoinositol | 25 g/l |
| Thiamine HCl | 0,25 g/l |
| Pyzidoxol HCl | 0,25 g/l |
| Acide p-aminobenzoïque | 0,05 g/l |

Composition de la solution de sels minéraux

| **Ingrédients** | **Concentration finale** |
|---|---|
| MgSO₄ | 26,76 g/l |
| CaCl₂ | 6,40 g/l |
| FeSO₄ | 5,61 g/l |
| CoCl₂ | 0,29 g/l |
| ZnSO₄ | 7,72 g/l |
| Na₂MoO₄ | 0,09 g/l |
| H₃BO₃ | 0,34 g/l |
| MnCl₂ | 0,47 g/l |
| CuSO₄ | 0,61 g/l |

Composition de la solution de FeSO₄

| **Ingrédients** | **Concentration finale** |
|---|---|
| FeSO₄ | 0,9 g/l |

Solution d'ammoniac, 14%
Solution anti-mousse : Struktol J673 diluée au 1/10 (Schill+Seilacher AG, Moorfleeter Str.28,22113, Hamburg, Allemagne)

### 2) Extraction d'ADN génomique et analyse Southern blot

### a) Extraction de l'ADN génomique

Le culot cellulaire obtenu à partir d'une culture de 4 ml est mis en suspension dans 0,5 ml de tampon sorbitol (0,9 M de sorbitol ; 0,1 M de Tris-HCl, pH = 8,0 ; 0,1 M d'EDTA). 50 µl de Zymolase® 20T (6 mg/ml) (Euromedéx, 67458 Mundolsheim cedex, France), 50 µl de 2-mercaptoéthanol à 0,28 M sont ajoutés et la solution est incubée 1 heure à 37°C sous agitation (180 rpm). La solution est soumise à centrifugation et le culot est mis en suspension dans 0,5 ml de tampon TE (50 mM de Tris-HCl, pH = 8; 20 mM d'EDTA). 50 µl de SDS 10% sont ajoutés et la solution est mélangée par retournements et incubée 20 min à 65°C. 0,2 ml d'acétate de potassium 5M sont ajoutés, puis la solution est mélangée et gardée dans la glace pendant 30 min, puis centrifugée pendant 5 min.

Le surnageant est transféré dans un tube de 1,5 ml puis 0,8 ml d'éthanol 100%, préalablement refroidi dans la glace, sont ajoutés. La solution est mélangée par retournements puis centrifugée. Après élimination du surnageant, 0,4 ml de tampon TE contenant 100 µg/ml de RNase A (Invitrogen, USA) sont ajoutés et la solution est incubée 1 heure à 37°C. Après l'ajout de 1 ml d'éthanol 100%, préalablement refroidi dans la glace, la solution est délicatement mélangée jusqu'à la précipitation de l'ADN, puis est centrifugée. Le surnageant est éliminé puis le culot d'ADN est séché à l'air libre puis mis en suspension dans 100 µl d'eau stérile puis incubé une nuit à 4°C.

### b) Digestion par l'enzyme HindIII

La concentration en ADN génomique est mesurée par détermination de l'absorbance à 260 nm (A₂₆₀) et à 280 nm (A₂₈₀). 1 µg de l'ADN génomique est mélangé avec de l'eau stérile jusqu'à un volume final de 42,5 µl. 5 µl de tampon (5X) et 2,5 µl de HindIII (50 u/µl) (Invitrogen, USA) sont ajoutés et la solution est incubée à 37°C pendant 4 heures.

### c) Analyse Southern Blot

Les transferts de type Southern blot sont effectués en suivant les procédures du kit « DIG High Prime DNA Labeling and Detection » de la société Roche Diagnostic.

### d) Procédure de marquage de la sonde

La sonde correspondant à la totalité du gène codant pour la lipase Lip2 est obtenue par PCR effectuée sur l'ADN génomique à l'aide de l'enzyme Phusion DNA polymerase (Finzyme) et des deux amorces suivantes :
Amorce sens : 5'-GTGTACACCTCTACCGAGACCTCT-3' (SEQ ID NO: 1)
Amorce antisens : 5'-TTAGATACCACAGACACCCTCGGT-3' (SEQ ID NO: 2)

Après la réaction de PCR, la sonde est purifiée sur gel à l'aide du kit « Nucleospin Extract II » (Macherey Nagel). La sonde purifiée est ensuite marquée à froid (digoxygénine) à l'aide du kit « DIG high prime DNA labeling » de Roche.

### e) Séparation sur gel, transfert d'ADN et détection du signal

2 µg de l'ADN digéré par HindIII est mélangé avec du tampon de charge puis est déposé sur un gel d'agarose 0,8%. La migration est effectuée sous 50 V pendant 2h30 puis l'ADN est transféré sur membrane Hybond+ (Amersham Bioscience). Le nombre de loci du gène *Lip2* dans l'ADN génomique est détecté grâce à l'hybridation, sur les membranes, de la sonde marquée, suivie de la visualisation par l'exposition aux rayons X.

### 3) Détermination de la concentration protéique

La concentration protéique est déterminée par la méthode de Bradford. Les protéines sont quantifiées à l'aide de la méthode de Bradford directement sur le surnageant de la culture en fermentation. La quantité de protéines ainsi déterminée est comparée avec des quantités connues de lipase Lip2 purifiée. 20 µl d'échantillons de standards, aux dilutions appropriées, sont mélangés dans des tubes avec 1 ml du réactif dilué (5 fois) contenant le colorant. La DO₅₉₅ est alors déterminée relativement à la DO₅₉₅ obtenue pour le contrôle (colorant dilué seul).

### 4) Mesure de l'activité spécifique de la lipase recombinante

L'activité de la lipase est mesurée potentiométriquement à 37°C à l'aide d'un dispositif pH-stat (RADIOMETER). Le substrat utilisé est la trioctanoïne. 10 mM de substrat sont mis en émulsion dans 15 ml de tampon de réaction contenant 1mM de Tris-HCl (pH 5,5), 150 mM de NaCl, 5 mM de CaCl₂ et 4 mM de taurodésoxycholate de sodium (SIGMA). Une unité d'activité spécifique est définie comme 1 µmole d'acide gras libéré par min et par mg de protéine.

### 4) Electrophorèse sur gel de polyacrylamide en condition dénaturante (SDS-PAGE)

12 µl d'un échantillon comprenant 25% d'un mélange contenant du SDS et des agents réducteurs sont chargés sur un gel bis tris 4-12% (Biorad). La migration est effectuée pendant 45 min à 160 V.

Le gel est ensuite analysé par coloration au bleu de Coomassie.

### 5) Détermination par spectrométrie de masse de type « Désorption-Ionisation Laser Assistée par Matrice - Temps de Vol » (MALDI-TOF : Matrix-assisted Desorption lonization-Time of Flight) de la masse moléculaire de la lipase Lip2 produite par le clone YL-LIP2-6C

Une analyse de spectrométrie de masse de type désorption/ionisation laser est effectuée en utilisant un spectromètre de masse « Voyager Elite XL time of flight » de la société Perseptive Biosystems (Framingham, MA) mis en oeuvre avec un laser à azote émettant à 337 nm. Le spectre de masse en mode positif est obtenu en utilisant un mode d'extraction linéaire et retardée avec un potentiel d'accélération de 25 kV, une tension de grille de 0,3%, une tension de guide d'ion de 0,3%, et un temps de retard de 1000 ns pour la protéine Lip2. Chaque spectre est le résultat de la moyenne de 100 impulsions laser. Le matériel à analyser est mélangé à un volume égal d'une solution saturée d'acide sinapinique (Fluka) préparé dans une solution à 50% (v/v) d'acétonitrile/acide trifluoroacétique aqueux. Des aliquots de 2µl de ce mélange sont déposés sur la plaque d'échantillon en acier inoxydable et sont séchés à l'air libre avant d'effectuer l'analyse. La calibration externe est effectuée à l'aide de l'alpha chymotrypsinogène A (Sigma). Les valeur exprimées sont des moyennes et correspondent à l'ion [M+H]⁺.

### EXEMPLE 2 - Construction du vecteur d'expression et obtention du clone YL-LIP2-6C

La souche YL-LIP2-6C est obtenue par transformation d'une souche de *Yarrowia lipolytica* POld, dépourvue de séquences zêta par un vecteur d'expression, dénommé JMP6, décrit dans la Demande EP 1 108 043, et comprenant le gène *LIP2* codant pour le précurseur Lip2p de la lipase Lip2 de *Yarrowia lipolytica* en amont duquel se trouve le promoteur ACO2. Ladite cassette est encadrée par des séquences zêta, comme décrit plus haut. La construction du vecteur JMP6 et la transformation de la souche POld sont réalisés selon le même mode opératoire que dans la Demande EP1 108043. Cette souche YL-LIP2-6C, comprenant 5 loci différents d'intégration de la cassette d'expression du gène *LIP2,* a été déposée auprès de la Collection Nationale de Cultures de Microorganismes (CNCM), 28 rue du Docteur Roux, 75724 Paris Cedex 15, sous le numéro I-3542, le 15 décembre 2005.

### EXEMPLE 3.- Production de la lipase avec la souche YL-LIP2-6C et vérification de la stabilité génétique de YL-LIP2-6C

La souche YL-LIP2-6C est mise en oeuvre dans un procédé de production de lipase comprenant une étape de pré-culture et une étape de fermentation adaptée à la production de lipase. L'approche globale de ce procédé est illustrée à la figure 1. La lipase ainsi produite a ensuite été analysée en terme d'activité et de qualité. Par ailleurs, la stabilité génétique de YL-LIP2-6C est analysée par détermination du nombre de loci de la cassette d'expression du gène *LIP2.*

### 1) Procédé de production

### Pré-cultures et fermentation

Une ampoule de solution-mère du clone YL-LIP2-6C dans du glycérol est décongelée et 5 µl sont mis en culture dans 25 ml de milieu 02130S enrichi, contenant 1‰ (v/v) de la solution de vitamines et 1‰ (v/v) de la solution d'oligoéléments, dans une bouteille Erlen Meyer de 250 ml. La culture est incubée à 28°C pendant 36 heures sous agitation (180 rpm). La préculture de 25 ml résultante (préculture 1) est ensemencée dans 200 ml de milieu 02130S enrichi dans un flacon Erlen Meyer de 2 litres puis la culture est incubée 36 heures à 28°C sous agitation (180 rpm). La préculture 2 ainsi obtenue (225 ml) est ensemencée dans du milieu 02130S enrichi dans un fermenteur.

Au temps To du procédé de fermentation, 2 ml de la solution de FeSO₄ sont ajoutés dans la culture. Toutes les 6 à 9 heures, 2 à 3 ml de la solution de vitamines sont ajoutés à la culture en fermentation et après 34 heures de fermentation (à T12), l'apport en glucose est initié. L'alimentation en glucose est progressivement augmentée pendant 14 heures puis est interrompue à T17 (correspondant à 48 heures de fermentation) et l'induction par l'acide oléique est alors initiée. L'alimentation en acide oléique est progressivement augmentée durant les 54 heures suivantes, puis à T33 (102 heures de fermentation) une DO₆₀₀ de 340 est atteinte et le procédé de fermentation est arrêté. La culture finale de 3 litres est centrifugée (14000 rpm). Le surnageant est récupéré et conservé à -20°C.

### Monitorage des cultures

Au cours de la fermentation, la température, la pression partielle en oxygène et le pH sont surveillés et maintenus à 28°C, 20% et 6,2 respectivement. Toutes les 3 heures environ, un échantillon de la culture est prélevé et la DO₆₀₀ est mesurée afin de suivre l'évolution du taux de croissance. Les résultats sont indiqués dans la figure 2. Deux extraits de 1 ml de ces échantillons sont centrifugés 5 min à 14000 rpm et les culots et surnageants sont conservés à -20°C. Le Tableau I montre le monitorage du procédé de fermentation.

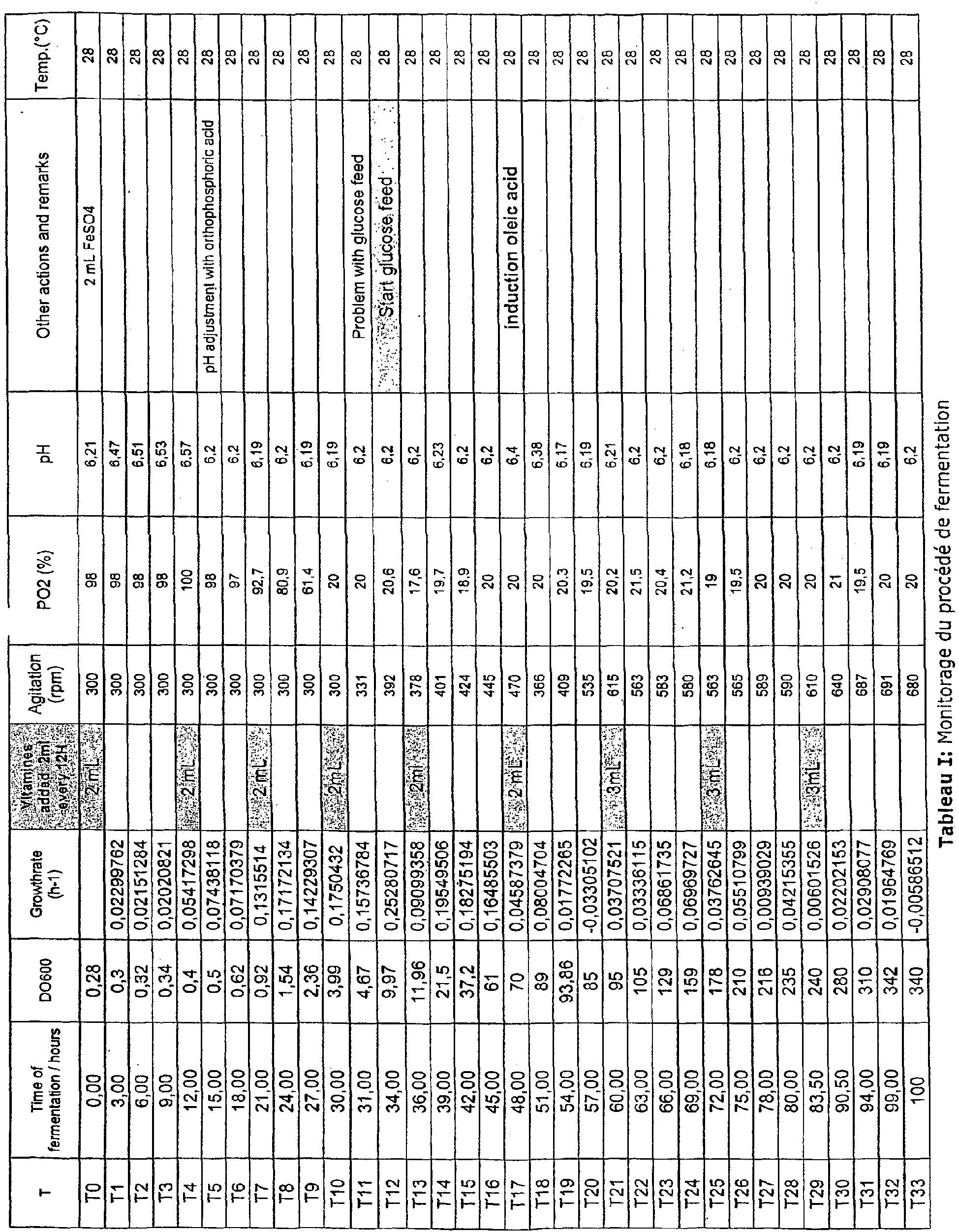

### Purification de la lipase

Optionnellement, une étape supplémentaire de purification de la lipase est réalisée.

La lipase est séparée de la culture à l'aide d'un dispositif de micro-filtration tangentielle sur membrane céramique (pilote X6 de PALL) permettant la rétention des levures de taille supérieure au seuil de coupure (0,1 µm). Le perméat contenant la lipase est récupéré d'abord en mode concentration, puis en mode diafiltration. Ces étapes sont mises en oeuvre en suivant les recommandations du fabricant avec les modifications appropriées. La concentration en microorganismes contenus dans le perméat est ensuite réduite par filtration (0,2 µm) (Millipore) de façon à obtenir une biocharge inférieure à 10 cfu/ml. A l'aide d'un dispositif Profux M12 (Millipore), la solution de lipase est concentrée, jusqu'à un volume d'environ 5 litres, et soumise à une ultrafiltration tangentielle en utilisant une membrane Pellicon standard Biomax 10 kDa afin d'éliminer les contaminants de faibles poids moléculaire. L'ultrafiltrat, ne contenant pas la lipase, est éliminé. La solution de lipase est alors une nouvelle fois purifiée par élimination des microorganismes, comme indiqué plus haut, de façon à obtenir une biocharge inférieure à 5 cfu/ml. La lipase ainsi purifiée peut en outre subir une lyophilisation.

### 3) Caractérisation de la lipase recombinante produite par YL-LIP2-6C

### a) Contrôle de la production de lipase par la souche YL-LIP2-6C au cours du procédé de fermentation

A chaque point de mesure, un échantillon de culture est soumis à centrifugation et le surnageant est analysé par SDS-PAGE. 75 µl du surnageant sont mélangés avec 75 µl d'eau puis 5 µl sont chargés sur un gel à 26 puits. Des échantillons contenant des quantités connues de lipase Lip2 sont également analysés. Les résultats sont illustrés à la figure 4. En comparant la quantité de lipase obtenue à la fin de la fermentation (T33), avec le gradient de quantités connues de lipase Lip2, la concentration de lipase obtenue après 100 heures de fermentation peut être estimée à 1,5 g/l.

Par ailleurs, les inventeurs ont mis en oeuvre le procédé de production de lipase selon l'invention pour un volume final d'environ 35 litres, ce qui a permis l'obtention de lipase selon un rendement situé entre 1 et 3 g par litre de surnageant de culture.

### b) Détermination de la concentration protéique et estimation du rondement de production de lipase recombinante

La concentration protéique dans le surnageant de culture est déterminée comme indiqué à l'exemple 1 (méthode de Bradford). Sept mesures indépendantes sont effectuées et la concentration de protéines dans le surnageant est de 2,3 g/l. La pureté de la protéine Lip2 dans le surnageant est estimée par SDS-PAGE. Les résultats sont illustrés à la figure 5. Le niveau de pureté est estimé à environ 70%. Le rendement résultant de production de lipase de l'étape de fermentation avec le clone YL-LIP2-6C est donc de 1,6 g/l.

### c) Mesure de l'activité spécifique de la lipase recombinante produite par YL-LIP2-6C

L'activité spécifique de la lipase produite par le clone YL-LIP2-6C est mesurée comme indiqué à l'exemple 1. L'échantillon correspondant au surnageant de fermentation est dilué 100 fois dans un tampon contenant 50 mM de Na₂HPO₄, 50mM de KH₂PO₄, 150 mM de NaCl, pH 6,0. L'activité catalytique déterminée à 37°C avec le trioctanoïne sur 3 expériences indépendantes est de 21883,33 U/ml du surnageant (Tableau II).

**Tableau II : mesure de l'activité d'échantillons du surnageant de fermentation**

| | Activité lipasique (U/ml) | | | | |
|---|---|---|---|---|---|
| | Essais | | | Moyenne | SD |
| Echantillon | 20800,00 | 22750,00 | 22100,00 | 21883,33 | 992,89 |

D'après la concentration estimée de lipase Lip2 dans le surnageant (voir plus haut), l'activité spécifique de la lipase est de 13675 U/mg, comparable à l'activité spécifique de la lipase produite pour les études cliniques. L'activité spécifique de la lipase produite par le clone YL-LIP2-6C est conforme aux conditions requises pour les lots cliniques.

### d) Masse moléculaire de la lipase recombinante

L'analyse du spectre de masse (voir exemple 1) est faite sur le surnageant de culture de fermentation après centrifugation sans purification. Le résultat est illustré à la figure 6. Le pic majeur observé révèle une masse moléculaire de 37648 Da. La lipase Lip2 produite par YL-LIP2-6C a une masse moléculaire correcte car la valeur observée est conforme aux conditions requises pour les lots cliniques, c'est à dire 37500 +/- 1000 Da.

Cet exemple illustre la sélection d'un clone stable, YL-LIP2-6C (en l'espèce, 100% des clones analysés après 30 générations présentent le même nombre de Loci du gène Lip2 que le clone initial). En outre, la stabilité génétique dudit clone n'est pas affectée par les conditions de culture mises en oeuvre pour la production de lipase (pendant les pré-cultures ; juste avant la fermentation ; juste avant l'induction par l'acide oléique de la production de lipase ; fin de la fermentation).

### 3) Stabilité génétique du clone YL-LIP2-6C

La stabilité génétique du clone YL-LIP2-6C au cours du procédé de production de lipase, comprenant une étape de préculture et une étape de fermentation, est analysée en déterminant le nombre d'évènements d'intégration de la cassette d'expression du gène *LIP2* à des loci différents dans l'ADN de *Yarrowia lipolytica,* dans les colonies sélectionnées à T0, T17 .et T33.

### a) Sélection des colonies

Un échantillon de la préculture 2 (T0, initiation de la fermentation), un échantillon de la culture en condition de fermentation à T17 (juste avant l'induction) et à T33 (fin de la fermentation) sont dilués, dans un premier temps jusqu'à atteindre une DO₆₀₀ = 1, puis sont dilués au 1/1000. 10 µl de ces solutions diluées sont chacune étalées sur 3 plaques de culture sur milieu YPD. Les plaques sont ensuite incubées à 28°C pendant 48 heures puis sont conservées à 4°C jusqu'à la sélection de colonies pour l'analyse du nombre de loci.

20 colonies dérivées de l'échantillon prélevé au point de départ de la fermentation (T0), 20 colonies dérivées de l'échantillon prélevé juste avant l'induction (T17) et 100 colonies dérivées de l'échantillon prélevé à la fin de la fermentation (T33) sont ensemencées séparément dans 4 ml de milieu 02130S enrichi puis mis en culture pendant 72 heures. Ensuite, des stocks dans du glycérol 30% sont constitués en prévision de l'extraction de l'ADN et de l'analyse par Southern blot (Matériels et Méthodes).

### c) Détermination du nombre d'évènements d'intégration du gène LIP2 à des loci différents pour 140 colonies dérivées de la souche YL-LIP2-6C

Les résultats de l'analyse par Southern blot du nombre d'événements d'intégration à des loci différents du gène *LIP2* sur 20 colonies prélevées au temps T0 (début de fermentation), 20 colonies prélevées au temps T17 (48 heures de fermentation) et 100 colonies prélevées au temps T33 (100 heures de fermentation) sont illustrés à la figure 3. Ces résultats montrent que toutes les colonies analysées contiennent 6 loci du gène *LIP2.* Dans la mesure où la souche sauvage contient une copie du gène *LIP2* endogène, la souche YL-LIP2-6C contient donc 5 loci d'intégrations de la cassette d'expression du gène *LIP2.* Le clone YL-LIP2-6C est donc 100% stable durant un procédé de fermentation de 100 heures.

## Revendications

1. Procédé de production de lipase recombinante, comprenant :
a) une étape au cours de laquelle on procède à la mise en culture de cellules de *Yarrowia lipolytica* transformées par un vecteur comprenant une cassette d'expression d'une lipase acido-résistante de levure, et
b) une étape au cours de laquelle on recueille, dans le surnageant de culture, la lipase recombinante produite par lesdites cellules,
lequel procédé est **caractérisé en ce que** l'étape a) de mise en culture est mise en oeuvre dans un milieu de culture dépourvu de produits d'origine animale ou de mélanges non caractérisés constitués de matières protéiques d'origine animale ou de leurs produits de digestion enzymatique
et **en ce que** ladite étape a) comprend :
a1) la pré-culture des cellules transformées de *Yarrowia lipolytica* dans un milieu contenant une source de carbone d'origine glucidique, de l'azote minéral et sels minéraux, des oligoéléments et des vitamines ; et
a2) une étape de fermentation, comprenant une phase de croissance cellulaire dans un milieu contenant comme seule source de carbone une source de carbone d'origine glucidique, de l'azote minéral et sels minéraux, des oligoéléments et des vitamines et une phase de production de lipase dans un milieu contenant comme seule source de carbone l'acide oléique, de l'azote minéral et sels minéraux, des oligoéléments et des vitamines.

2. Procédé de production de lipase recombinante selon la revendication 1, **caractérisé en ce que** ladite source d'azote est le sulfate d'ammonium.

3. Procédé de production selon la revendication 1 ou 2, **caractérisé en ce que** la fermentation est avantageusement mise en oeuvre avec une pO2 constante comprise entre 15 % et 25 % et un pH de préférence inférieur à 6,5.

4. Procédé de production selon la revendication 2 ou la revendication 3, **caractérisé en ce que** l'étape a1) de pré-culture est réalisée jusqu'à atteindre une valeur de DO₆₀₀ₙₘ comprise entre 3 et 10 pour 1 ml, et **en ce qu'**à l'étape a2) de fermentation, ladite phase de production de lipase est initiée lorsque la DO₆₀₀ₙₘ de la culture atteint une valeur comprise entre 60 et 80 pour 1 ml.

5. Procédé de production selon l'une quelconque des revendications 1 à 4, **caractérisé en ce que** l'étape b) au cours de laquelle on recueille la lipase, est réalisée lorsque la DO₆₀₀ₙₘ atteint une valeur comprise entre 300 et 350 pour 1 ml.

6. Procédé de production selon l'une quelconque des revendications 1 à 5, **caractérisé en ce que** l'étape b) comprend :
b1) la séparation de la lipase dudit surnageant de culture, et
b2) la purification de la lipase obtenue en b1).

7. Procédé de production selon la revendication 6, **caractérisé en ce que** ladite séparation est effectuée par une technique choisie parmi la filtration tangentielle en fibre creuse, la filtration frontale et la centrifugation continue ou discontinue, et ladite purification est effectuée par une technique choisie parmi la filtration, la précipitation fractionnée, la chromatographie d'échange d'ions, la chromatographie d'interactions hydrophobes et la chromatographie par gel-filtration.

8. Procédé de production selon l'une quelconque des revendications 1 à 7, **caractérisé en ce que** l'étape a) consiste en la mise en culture du clone dénommé YL-LIP2-6C, déposé auprès de la Collection Nationale de Cultures de Microorganismes (C.N.C.M.), 28 rue du Docteur Roux, 75724 Paris Cedex 15, sous le numéro I-3542, le 15 décembre 2005.

9. Préparation de la lipase recombinante acido-résistante de levure codée par le gène Lip2 ou LIP2, **caractérisée en ce qu'**elle est susceptible d'être obtenue par un procédé selon la revendication 8, **en ce qu'**elle a une activité catalytique à pH6 d'au moins 15000 unités par ml de surnageant de culture, de préférence supérieure à 20000 unités par ml de surnageant de culture, une unité correspondant à la quantité d'enzyme capable de catalyser la libération de 1 µmole d'acide gras par minute lorsque le substrat utilisé est la trioctanoïne, et **en ce que** la concentration de ladite lipase dans ladite préparation est supérieure à 1 g de lipase par litre.

10. Utilisation d'une préparation de lipase selon la revendication 9 pour l'obtention d'un médicament destiné au traitement d'un syndrome de malabsorption de graisses, lié à une insuffisance pancréatique.

11. Préparation de lipase selon la revendication 9 pour une utilisation comme médicament.

12. Cellule de *Yarrowia lipolytica* transformée par un vecteur comprenant une cassette d'expression d'une lipase extracellulaire acido-résistante de levure, **caractérisée en ce qu'**il s'agit du clone dénommé YL-LIP2-6C, déposé auprès de la Collection Nationale de Cultures de Microorganismes (C.N.C.M.) sous le numéro I-3542, le 15 décembre 2005.

13. Utilisation de la cellule selon la revendication 12 pour la production de la lipase acido-résistante de levure codée par le gène Lip2 ou LIP2.

## Patentansprüche

1. Ein Verfahren zur Herstellung rekombinanter Lipase, umfassend:
a) einen Schritt, bei dem man *Yarrowia lipolytica-Zellen* kultiviert, die mit einem Vektor transformiert sind enthaltend eine Expressionskassette einer säurebeständigen Hefe-Lipase, und
b) einen Schritt, bei dem man in dem Kulturüberstand die durch die Zellen hergestellte rekombinante Lipase gewinnt,
wobei das Verfahren **dadurch gekennzeichnet ist, dass** der Schritt a) der Kultivierung in einem Kulturmedium durchgeführt wird, das frei ist von Produkten tierischen Ursprungs oder von nicht charakterisierten Mischungen bestehend aus proteinhalti-gen Materialien tierischen Ursprungs oder ihren enzymatischen Abbauprodukten; und dadurch, dass der Schritt a) umfasst:
a1) die Vorkultivierung der transformierten *Yarrowia lipolytica-Zellen* in einem Medium, das eine auf Kohlenhydrate basierende Kohlenstoffquelle, anorgani-schen Stickstoff und anorganische Salze, Spurenelemente und Vitamine aufweist; und
a2) einen Fermentationsschritt, umfassend eine Zellwachstumsphase in einem Medium enthaltend eine auf Kohlenhydrate basierende Kohlenstoffquelle als einzige Kohlenstoffquelle, anorganischen Stickstoff und anorganische Salze, Spurenelemente und Vitamine, und eine Produktionsphase der Lipase in einem Medium enthaltend Ölsäure als einzige Kohlenstoffquelle, anorganischen Stickstoff und anorganische Salze, Spurenelemente und Vitamine.

2. Das Verfahren zur Herstellung rekombinanter Lipase gemäß Anspruch 1, **dadurch gekennzeichnet, dass** die Stickstoffquelle Ammoniumsulfat ist.

3. Das Herstellungsverfahren gemäß Anspruch 1 oder 2, **dadurch gekennzeichnet, dass** die Fermentation vorteilhafterweise mit einem konstanten p02 zwischen 15% und 25% und einem pH-Wert vorzugsweise unter 6,5 durchgeführt wird.

4. Das Herstellungsverfahren gemäß Anspruch 2 oder Anspruch 3, **dadurch gekennzeichnet, dass** der Schritt a1) der Vorkultivierung durchgeführt wird bis ein OD₆₀₀ₙₘ-Wert zwischen 3 und 10 für 1 ml erreicht wird, und dadurch, dass in Schritt a2) der Fermentation, die Produktionsphase der Lipase initiiert wird, wenn der OD₆₀₀ₙₘ-Wert der Kultur einen Wert zwischen 60 und 80 für 1 ml erreicht.

5. Das Herstellungsverfahren gemäß irgendeinem der Ansprüche 1 bis 4, **dadurch gekennzeichnet, dass** der Schritt b), bei dem die Lipase gewonnen wird, durchgeführt wird, wenn der OD₆₀₀ₙₘ-Wert einen Wert zwischen 300 und 350 für 1 ml erreicht.

6. Das Herstellungsverfahren gemäß irgendeinem der Ansprüche 1 bis 5, **dadurch gekennzeichnet, dass** der Schritt b) umfasst:
b1) die Abtrennung der Lipase aus dem Kulturüberstand, und
b2) die Aufreinigung der in b1) erhaltenen Lipase.

7. Das Herstellungsverfahren gemäß Anspruch 6, **dadurch gekennzeichnet, dass** die Trennung durch eine Technik erreicht wird ausgewählt aus der Tangentialflussfiltration über Hohlfasern, der Frontalfiltration und der kontinuierlichen oder diskontinuierlichen Zentrifugation, und die Aufreinigung durchgeführt wird durch eine Technik ausgewählt aus der Filtration, der fraktionierten Fällung, der lonenaustauschchromatographie, der hydrophoben Interaktionschromatographie und der Chromatographie über Gelfiltration,

8. Das Herstellungsverfahren gemäß irgendeinem der Ansprüche 1 bis 7, **dadurch gekennzeichnet, dass** der Schritt a) auf der Kultivierung des Klons mit der Bezeichnung YL-LIP2-6C beruht, der bei der Collection Nationale de Cultures de Microorganismes (C.N.C.M.), 28 rue du Docteur Roux, 75724 Paris Cedex 15, unter der Nummer I-3542 am 15. Dezember 2005 hinterlegt wurde.

9. Zubereitung einer rekombinanten säurebeständigen Hefe-Lipase, wobei die Lipase von dem Gen Lip2 oder LIP2 kodiert wird, **dadurch gekennzeichnet, dass** sie durch ein Verfahren nach Anspruch 8 erhältlich ist, und dadurch, dass sie eine katalytische Aktivität bei pH 6 von mindestens 15.000 Einheiten pro ml Kulturüberstand, vorzugsweise mehr als 20.000 Einheiten pro ml Kulturüberstand aufweist, wobei eine Einheit der Menge eines Enzyms entspricht, die in der Lage ist, die Freisetzung von 1 µMol Fettsäure pro Minute zu katalysieren, wenn das verwendete Substrat Glycerintrioctanoat ist, und dadurch, dass die Konzentration der Lipase in der Zubereitung mehr als 1 g Lipase pro Liter beträgt.

10. Verwendung einer Lipase-Zubereitung gemäß Anspruch 9 zur Bereitstellung eines Medikaments für die Behandlung eines Fett-Malabsorptionssyndroms, welches mit einer Pankreasinsuffizienz verbunden ist.

11. Lipase-Zubereitung gemäß Anspruch 9 zur Verwendung als Arzneimittel.

12. *Yarrowia lipolytica-Zelle,* welche mit einem Vektor transformiert ist, enthaltend eine Expressionskassette für eine extrazelluläre säurebeständige Hefe-Lipase, **dadurch gekennzeichnet, dass** es sich um den Klon mit der Bezeichnung YL-LIP26C handelt, der bei der Collection Nationale de Cultures de Microorganismes (C.N.C.M.), 28 rue du Docteur Roux, 75724 Paris Cedex 15, unter der Nummer I-3542 am 15. Dezember 2005 hinterlegt wurde.

13. Verwendung der Zelle gemäß Anspruch 12 zur Herstellung der säurebeständigen Hefe-Lipase, die von dem Gen Lip2 oder LIP2 kodiert wird.

## Claims

1. Method for production of recombinant lipase, comprising:
a) a step of culturing Yarrowia lipolytica cells transformed with a vector comprising a cassette for expressing a yeast acid-resistant lipase, and
b) a step of collecting the recombinant lipase produced by said cells in the culture supernatant, which method is **characterised in that** said culturing step a) is carried out in a culture medium devoid of products of animal origin or an uncharacterised mixtures of protein materials of animal origin or of products of their enzymatic digestion,
and **in that** said step a) comprises:
a1) pre-culturing the transformed cells of Yarrowia lipolytica in a medium containing a sugar-based carbon source, mineral nitrogen and mineral salts, oligoelements and vitamins; and
a2) a fermentation step, comprising a cell growth phase in a medium containing as the sole carbon source a sugar-based carbon source, mineral nitrogen and mineral salts, oligoelements and vitamins and a lipase production phase in a medium containing as the sole carbon source oleic acid, mineral nitrogen and mineral salts, oligoelements and vitamins.

2. Recombinant lipase production method according to claim 1, **characterised in that** said nitrogen source is ammonium sulphate.

3. Production method according to claim 1 or 2, **characterised in that** the fermentation is advantageously carried out with a constant pO2 between 15% and 25% and a pH preferably below 6.5.

4. Production method according to claim 2 or claim 3, **characterised in that** the pre-culturing step a1) is carried out until a DO₆₀₀ₙₘ value between 3 and 10 per 1 ml is reached, and **in that** in the fermenting step a2) said lipase production phase is initiated when the DO₆₀₀ₙₘ of the culture reaches a value between 60 and 80 per 1 ml.

5. Production method according to any one of claims 1 to 4, **characterised in that** the step b) in which the lipase is recovered is carried out when the DO₆₀₀ₙₘ reaches a value between 300 and 350 per 1 ml.

6. Production method according to any one of claims 1 to 5, **characterised in that** the step b) comprises:
b1) separating the lipase from said culture supernatant, and
b2) purifying the lipase obtained in b1).

7. Production method according to claim 6, **characterised in that** said separation is effected by a technique selected from hollow fiber tangential filtration, frontal filtration and continuous or discontinuous centrifugation and said purification is performed by a technique selected from filtration, fractional precipitation, ion exchange chromatography, hydrophobic interaction chromatography and gel-filtration chromatography.

8. Production method according to any one of claims 1 to 7, **characterised in that** the step a) comprises culturing the clone YL-LIP2-6C deposited with the Collection Nationale de Cultures de Microorganismes (C.N.C.M.), 28 rue du Docteur Roux, 75724 Paris Cedex 15, under the number 1-3542, on 15 December 2005.

9. Preparation of yeast acid-resistant recombinant lipase, **characterised in that** it is obtainable by a method according to claim 8 and **in that** it has a catalytic activity at pH6 of at least 15000 units per ml of culture supernatant, preferably greater than 20000 units per ml of culture supernatant, a unit corresponding to the amount of enzyme capable of catalyzing the release of 1 µmol of fatty acid per minute when the substrate used is trioctanoin and **in that** the concentration of said lipase in said preparation is greater than 1 g of lipase per litre.

10. Use of a lipase preparation according to claim 9 for obtaining a medicament for the treatment of a fat malabsorption syndrome linked to a pancreatic insufficiency.

11. Lipase preparation according to claim 9 for use as a medicament.

12. Yarrowia lipolytica cell transformed with a vector comprising a cassette for expressing an extracellular yeast acid-resistant lipase, **characterised in that** it is the clone YL-LIP2-6C deposited with the Collection Nationale de Cultures de Microorganismes (C.N.C.M.) under the number 1-3542 on 15 December 2005.

13. Use of the cell according to claim 12 for the production of yeast acid-resistant lipase coded by the gene Lip2 or LIP2.


### SEQUENCE LISTING

<110> LABORATOIRES MAYOLY SPINDLER
   LEBLOND, Yves
   MOUZ, Nicolas
<120> procédé de production de lipase, cellule de Yarrowia lipolytica transformée apte à produire ladite lipase et leurs applications.
<130> F269Cas39PCT
<160> 2
<170> PatentIn version 3.3
<210> 1
   <211> 24
   <212> DNA
   <213> artificial sequence
<220>
   <223> amorce sens
<400> 1
   gtgtacacct ctaccgagac ctct 24
<210> 2
   <211> 24
   <212> DNA
   <213> artificial sequence
<220>
   <223> amorce antisens
<400> 2
   ttagatacca cagacaccct cggt 24